# EUROPÄISCHE PATENTSCHRIFT

(11) **EP 1 294 291 B1**
(45) Veröffentlichungstag und Bekanntmachung des Hinweises auf die Patenterteilung: **25.08.2004**
(21) Anmeldenummer: 01951641.8
(22) Anmeldetag: 28.06.2001
(51) Int. Cl.: A61B 17/115

(54) **GLEITSCHUTZ FUR EINEN GEHÄUSEKOPF MEDIZINISCHER INSTRUMENTE**
ANTI-SLIP PROTECTION DEVICE FOR A HOUSING HEAD FOR MEDICAL INSTRUMENTS
DISPOSITIF ANTIDERAPANT POUR TETE DE BOITIER D'INSTRUMENT MEDICAL

(30) Priorität: 28.06.2000 DE 10031436
(43) Veröffentlichungstag der Anmeldung: 26.03.2003
(73) Patentinhaber: Von Fuchs, Alexander, 5026 Salzburg (AT)
(72) Erfinder: Von Fuchs, Alexander, 5026 Salzburg (AT)
(74) Vertreter: Gallo, Wolfgang, Dipl.-Ing. (FH)
(86) Internationale Anmeldenummer: PCT/EP2001/007375
(87) Internationale Veröffentlichungsnummer: WO 2002/000121

(56) Entgegenhaltungen:
- DE-U- 29 914 859
- US-A- 3 513 830
- US-A- 4 817 847
- US-A- 5 129 402
- US-A- 5 197 648
- US-A- 5 404 870

## Beschreibung

Die vorliegende Erfindung betrifft einen Gleitschutz für einen Gehäusekopf medizinischer Instrumente, insbesondere intraluminaler für Anastomosen nach Darmresektionen.

Obwohl auf beliebige Darmanastomosen anwendbar, werden die vorliegende Erfindung sowie die ihr zugrunde liegende Problematik in Bezug auf Resektionsoperationen im Bereich des Mastdarms beschrieben.

Bei Darmresektionsoperationen aufgrund von gut- oder bösartigen Tumoren im Bereich des Colon Descendens bzw. des Colon Sigmoideums oder des Rectrums wird der den Tumor tragende Darmabschnitt reseziert, die beiden diskonektierten Darmabschnitte werden zusammengeführt und mittels einer Naht, beispielsweise einer Tabaksbeutelnaht verbunden.

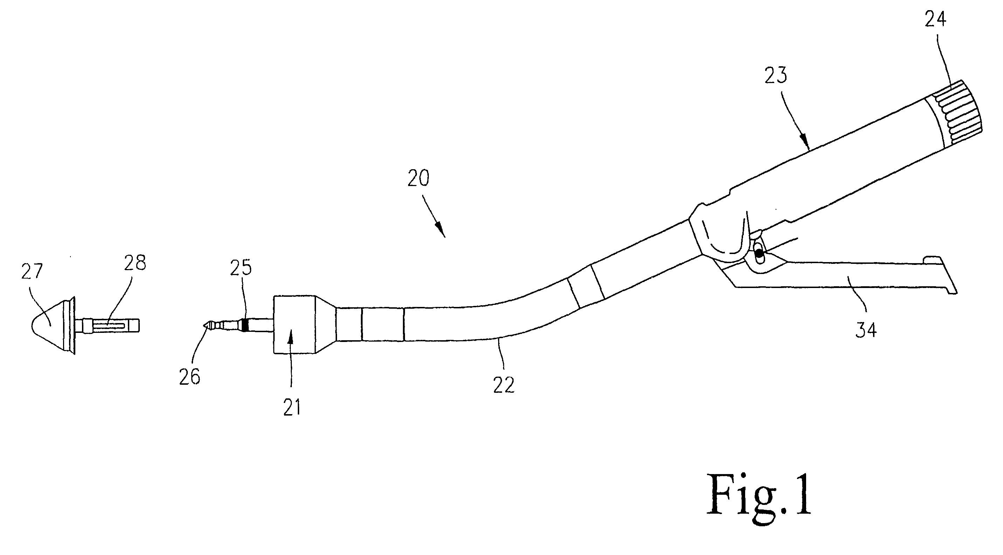

Dieser Vorgang wird überlicherweise durch ein sogenanntes intraluminales zirkuläres Klammernahtinstruments, wie es in der Fig. 1 ersichtlich ist, durchgeführt.

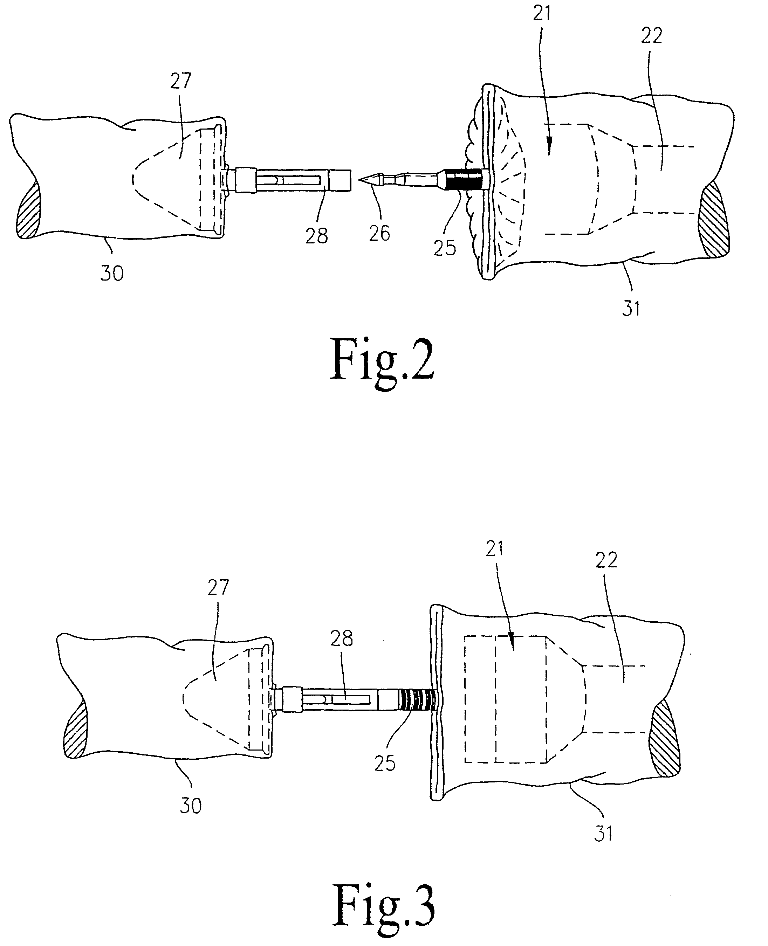

Ein solcher Operationsvorgang soll mit Hilfe der Figuren 1 bis 5 im Folgenden beschrieben werden. Eine Andruckeinrichtung 27, die eine Aufnahmehülse 28 aufweist, wird in den proximalen Darmabschnitt 30, wie in Figur 2 dargestellt, eingeknotet. Durch den Anus wird das entsprechende Gegenstück, bestehend aus einem Gehäuseschaft 22, einem Griff 23 mit einer Dreheinrichtung 24, und aus einem Gehäusekopf 21, aus dem ein Verbindungsstift 25 mittels der Dreheinrichtung 24 in Richtung der Symmetrieachse des Gehäuseschachts 22 ausfahrbar ist, eingeführt.

Ferner ist im Gehäusekopf 21 ein Schneid- und Klammermechanismus 29 vorgesehen, der durch einen am Griff 23 angeordneten Auslösehebel 34 betätigbar ist.

Nachdem ein Operateur den Gehäuseschacht 22 mit dem Gehäusekopf 21 durch den Enddarm eingefädelt hat, wird der Verbindungsstift 25 mittels der Dreheinrichtung 24 ausgefahren, wobei sich eine Spitze 26 des Verbindungsstiftes 25 durch die Mitte geklammerten distalen Bahnabschnitts 31 bohrt, wie in der Fig. 2 dargestellt.

Als nächstes wird, wie die Fig. 3 zeigt, die Aufnahmehülse 28 der Andruckeinrichtung 27 auf den Verbindungsstift 25 formschlüssig aufgesteckt, wobei eine Federeinrichtung für einen festen Kontakt dient.

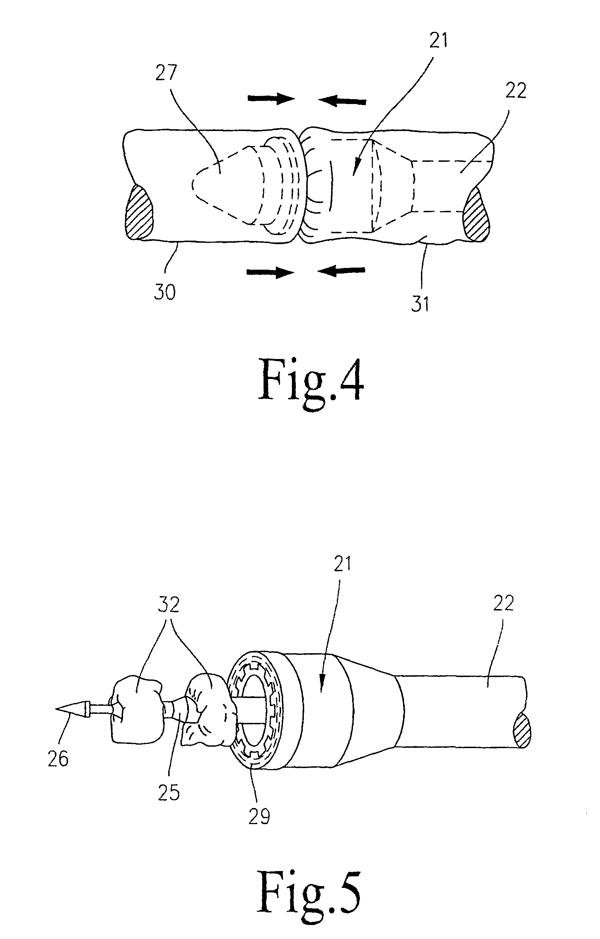

Danach werden, wie in Fig. 4 dargestellt, die Andruckeinrichtung 27 und der Gehäusekopf 21 durch ein Zurückfahren des Verbindungsstiftes 25 in den Gehäusekopf 21 mittels der Dreheinrichtung 24 zusammengedrückt, und nach Erreichen einer markierten Stellung wird durch den Operateur der Schneid- und Klammermechanismus durch Drücken des Auslösehebels 34 ausgelöst.

Fig. 5 zeigt das Ergebnis dieses Operationsvorgangs, bei dem jeweils ein Darmring bestimmter Länge sowohl aus dem proximalen Darmabschnitt 30 als auch aus dem distalen Darmabschnitt 31 herausgeschnitten und zusammen mit dem intraluminalen Instrument 20 durch den Anus entfernt ist.

Die der vorliegenden Erfindung zugrunde liegende Problematik besteht darin, dass es bei einer großen Anzahl von Patienten bei dem oben beschriebenen Operationsverfahren postoperativ zu Schwierigkeiten kommt, den Schließmuskel willkürlich zu betätigen. Somit können diese Patienten ihren Stuhlgang nicht richtig beherrschen.

Es gibt hauptsächlich zwei Gründe für das Auftreten dieser Schwierigkeiten. Zum einen entstehen durch das Vorschieben des Gehäusekopfes 21 in dem Darm aufgrund der vorderen Umfangskante häufig Einrisse und Ausdünnungen der Muskulatur des Sphinkter Internus, und seltenen des Sphinkter externus.

Außerdem wird die weiche Mucosa des Darms, also die Darmschleimhaut, im Inneren des Darmabschnitts durch den Gehäusekopf beim Vorschieben des intraluminalen Instruments in der Ausnehmung des Gehäusekopfes mitgeschoben. Dabei wird die Darmschleimhaut über die Maße gestreckt und am Anastomosebereich fixiert. Die weiche Darmschleimhaut besitzt die Tendenz, sich in die ursprüngliche Lage zurückzuziehen. Durch diese Dehnungstendenz kann eine lokale Minderperfusion, also eine zu geringe Durchblutung, im Anastomosenbereich entstehen. Diese Minderdurchblutung kann zu Anastomoseninsufizienzen führen, die eine schwere Komplikation für den Patienten darstellen.

Daneben kann es zu Ausdünnungen und Zerreißungen der Darmserosa kommen, die ebenfalls zur erheblichen Komplikationen führen.

Somit ist es Aufgabe der vorliegenden Erfindung, einen Gleitschutz für einen Gehäusekopf medizinischer Instrumente zu schaffen, der das Einführen und Vorschieben dieser Instrumente durch den Darm ohne eine Verletzung und ein Mitschieben der Darmschleimhaut ermöglicht und der den weiteren obenbeschriebenen Operationsvorgang nicht behindert.

Gemäß der vorliegenden Erfindung besteht der Gleitschutz aus einer am Gehäusekopf überkragend befestigbaren Abschlussvorrichtung, die fernbetätigbar in wenigstens zwei Abschnitten vom Gehäusekopf entfernbar ist.

Der erfindungsgemäße Gleitschutz mit den Merkmalen des Anspruchs 1 weist gegenüber dem Stand der Technik den Vorteil auf, dass sowohl die Ausnehmung als auch die Umfangskante des Gehäusekopfs durch die Abschlussvorrichtung bedeckt sind, wodurch ein Mitschieben der Darmschleimhaut und eine Verletzung der Muskulatur bzw. der Darmserosa verhindert wird. Außerdem kann der Gleitschutz beim Erreichen des zu anastomisierenden Darmabschnitt fernbetätigbar durch den Operateur vom Gehäusekopf entfernt werden. Somit steht der Gleitschutz den anschließenden Vorgängen, beispielsweise dem Schneiden und Klammern, nicht im Wege.

In den Unteransprüchen finden sich vorteilhafte Weiterbildungen und Verbesserungen des in Anspruch 1 angegebenen Gleitschutzes.

Gemäß einer bevorzugten Weiterbildung ist die Abschlussvorrichtung rund, insbesondere kegel- bzw. eiförmig, mit einem einseitig abgeflachten Ende ausgebildet. Durch diese Rundform kann der Reibungskoeffizient erheblich verringert und Verletzungen im Inneren des Darms vermieden werden.

Gemäß einer weiteren bevorzugten Weiterbildung ist die Abschlussvorrichtung aus einem elastisch verformbaren Material hergestellt. Somit stellt sich einerseits kein Verletzungsrisiko im Inneren des Darms dar, und andererseits kann sie an Gehäuseköpfen mit verschiedenen Durchmessern angebracht werden, die beim derzeitigen Stand der zur Verfügung stehenden Instrumentariums von 25 bis 33 mm variiert.

Gemäß einer weiteren bevorzugten Weiterbildung weist die Abschlussvorrichtung an jedem der wenigstens zwei Abschnitte mindestens eine Zugeinrichtungs-Befestigungseinrichtung auf, an der eine fernbetätigbare Zugeinrichtung, insbesondere eine reißfeste Schnur anbringbar ist. Dadurch ist kostengünstig eine Möglichkeit geschaffen, den Gleitschutz fernbetätigbar vom Gehäusekopf zu lösen und extraluminal aus dem Darm zu entfernen.

Gemäß einer weiteren bevorzugten Weiterbildung ist die Zugeinrichtungs-Befestigungseinrichtung als versenkte Befestigungsöse ausgebildet.

Gemäß einer weiteren bevorzugten Weiterbildung sind die wenigstens zwei Abschnitte symmetrisch ausgebildet. Dies bietet den Vorteil, dass nicht ein unnötig großer Abschnitt zwischen dem medizinischen Instrument und dem dehnbaren Darm herausgezogen werden muss.

Gemäß einer weiteren bevorzugten Weiterbildung sind die wenigstens zwei Abschnitte über Verbindungseinrichtungen, insbesondere als Steckbrücken ausgebildete Sollbruchstellen, miteinander verbindbar. Somit sind die beiden Abschnitte auf kostengünstig und einfach herstellbare Weise miteinander verbindbar und mittels einer bestimmten Kraft leicht trennbar.

Gemäß einer weiteren bevorzugten Weiterbildung weisen die wenigstens zwei Abschnitte jeweils flache Bereiche auf, die bei einer Verbindung der wenigstens zwei Abschnitte abschließend aneinanderliegen. Diese Bereiche ermöglichen einen einfachen für eine Trennung notwendigen Kraftübertrag.

Gemäß einer weiteren bevorzugten Weiterbildung weist jeder der wenigstens zwei Abschnitte am abgeflachten Ende mindestens einen Anlagefuß auf, der abschließend teilweise zum äußeren Umfang des Gehäusekopfes umschließt. Diese Anlagefüßen dienen zur zusätzlichen Führung der Abschlussvorrichtung auf dem Gehäusekopf.

Gemäß einer weiteren bevorzugten Weiterbildung sind die Anlagefüße elastisch verformbar ausgebildet. Somit schließen sie auch bei unterschiedlichen Gehäusekopfdurchmessern optimal mit dem äußeren Umfang des entsprechenden Gehäusekopfes ab.

Gemäß einer weiteren bevorzugten Weiterbildung weist das abgeflachte Ende der Abschlussvorrichtung einen hohlzylindrisch ausgebildeten Aufsteck-Anlageabschnitt auf. Dieser dient einem einfachen Aufstecken der Abschlussvorrichtung auf einen Gehäusekopf. Da sowohl die gesamte Abschlussvorrichtung als auch die Behandlung des Aufsteck-Anlageabschnitts aus einem elastisch verformbaren Material ausgebildet ist, wird ein einfaches Aufstecken und Halten auf Gehäuseköpfe mit unterschiedlichen Durchmessern gewährleistet.

Gemäß einer weiteren bevorzugten Weiterbildung weist der Aufsteck-Anlageabschnitt eine kreisförmige Anlagefläche mit einer mittig angeordneten, konisch ausgebildeten Spreizstift-Führungsausnehmung auf. Durch die konische Ausbildung wird eine Einführung des aus dem Gehäusekopf ausfahrenden Verbindungsstiftes in die Spreizstift-Führungsausnehmung vereinfacht. Dadurch wird die Trennung der beiden Abschnitte durch eine Krafteinwirkung des ausfahrenden Verbindungsstiftes ermöglicht.

In einer weiteren Ausgestaltung der Erfindung sind die beiden Abschnitte des Gleitschutzes als abgerundete Kappenteile ausgebildet und miteinander über lösbare Zugbandbefestigungen verbunden. Dabei ist vorzugsweise das Ende des Bandes an jedem Kappenteil separat befestigt, wobei das Zugband zwischen seiner endseitigen Befestigung und der die Kappen verbindenden Befestigung wenigstens einmal annähernd rechtwinklig gegen die Innenfläche des Kappenteils gefaltet ist. Vorteilhaft wird damit zum einen durch die lösbare Zugbandverbindung eine lösbare Verbindung der Kappenteile verwirklicht, die mit dem Zugband selbst gebildet ist, wobei das Zugband dann bevorzugt nach zweifacher annähernd rechtwinkliger Faltung gegen die Innenfläche des Kappenteils zu einem Endbefestigungspunkt geführt ist, der senkrecht zur Trennungslinie der Kappenteile liegt.

Wenn zu dem vorteilhaft vorgesehen ist, dass sich der Kappenteil einer randseitige Zugband-Führungseinformung nahe seiner endseitigen Befestigung aufweist, lassen sich günstiger Weise für das Entfernen für jedes Kappenteil definierte Zugrichtungen herstellen, wobei die Führungseinformung vorteilhaft in eine bevorzugt für jedes Kappenteil vorgesehene randseitige Anlageeinformung für den Gehäusekopf eingearbeitet ist.

Aufgrund dieser Ausgestaltung ist ein einfaches Trennen der Kappenteile und Entfernen nach Entfalten des zusammengelegten Bandes und damit der Freigabe der Verbindungsstelle zwischen den Kappenteilen ermöglicht.

Ausführungsbeispiele der Erfindungen sind in den Zeichnungen dargestellt und in der nachfolgenden Beschreibung näher erläutert.

Es zeigen:
- Fig. 1: eine Seitenansicht eines intraluminalen Instruments mit abgeschraubter Andruckeinrichtung;
- Fig. 2 bis 5: perspektivische Ansichten eines Resektion- oder Anastomosevorgangs mittels dem intraluminalen Instrumtes aus Fig.1;
- Fig. 6: eine Vorderansicht eines Gleitschutzes, teilweise im Schnitt, gemäß einem Ausführungsbeispiel der vorliegenden Erfindung;
- Fig. 7: eine Unteransicht des Gleitschutzes aus Fig. 6;
- Fig. 8: eine schematische Ansicht eines auf einem intraluminalen Instrument angebrachten Gleitschutzes beim Einführen in einen Darmabschnitt;
- Fig. 9: schematisch einen Trenn- und Entfemvorgang eines Gleitschutzes von einem Gehäusekopf gemäß einem Ausführungsbeispiel der vorliegenden Erfindung;
- Fig. 10: ein weiteres Ausführungsbeispiel eines Gleitschutzes mit eiförmigen Kappenteilen und Zugbändern in Anordnung auf einem Gehäusekopf;
- Fig. 11: eine Innenansicht des Gleitschutzes gemäß Fig. 10 zur Darstellung der endseitigen Bandbefestigung und der kappenverbindenden Befestigung durch die Zugbänder; und

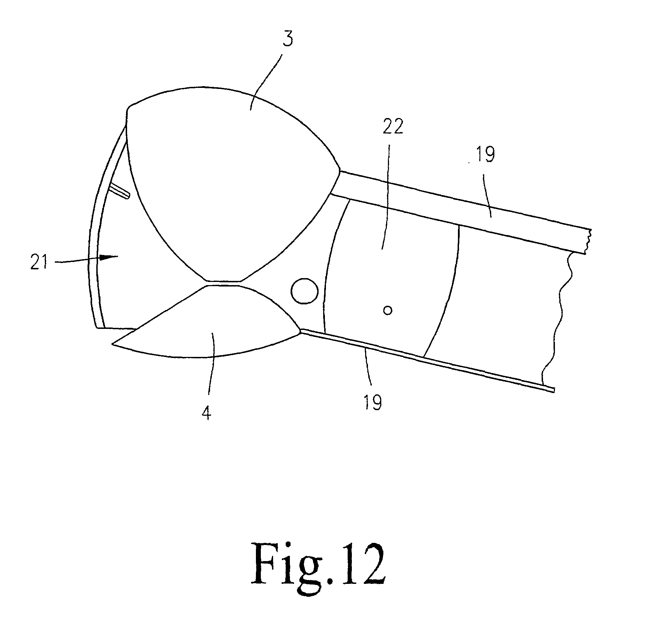

- Fig. 12: eine Seitenansicht auf den Gehäusekopf gemäß Fig. 4 in der ersten Phase nach Öffnen der Kappenteile.

In den Figuren bezeichnen gleiche Bezugszeichen gleiche oder funktionsgleiche Komponenten.

Die Figuren 1 bis 5 zeigen ein intraluminales Instrument 20 und die einzelnen Schritte einer mit diesen Instrument ausgeführten Resektionsoperationen. Diese Figuren sind bereits in der Beschreibungseinleitung ausführlich beschrieben.

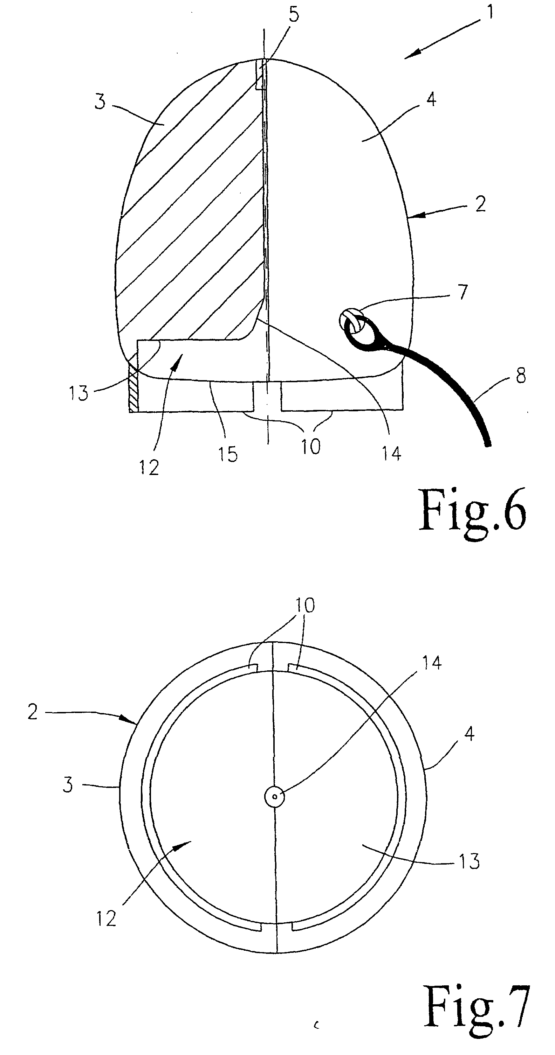

Fig. 6 zeigt eine Vorderansicht eines Gleitschutzes 1 gemäß einem Ausführungsbeispiel der vorliegenden Erfindung, wobei die linke Hälfte im Schnitt dargestellt ist.

Der in Fig. 1 dargestellte Gleitschutz 1 besteht auf einer Abschlussvorrichtung 2 mit zwei Abschnitten 3, 4, die an ihrer äußeren Verbindungslinie vorteilhaft mittels stegförmigen Steckbrücken 5 miteinander verbunden sind.

Die Abschlussvorrichtung 2 ist gemäß diesem Ausführungsbeispiel eiförmig mit einem einseitig abgeflachten Ende 15 ausgebildet. An diesem abgeflachten Ende 15 ist pro Abschnitt 3, 4 jeweils ein Anlagefuß 10 in Form einer halbringförmigen Manschette angeformt.

Sowohl die Abschlussvorrichtung 2 als auch die Anlagefüße 10 bestehen aus einem elastischen verformbaren Material, vorteilhaft aus einem elastisch verformbaren Kunststoff. Die beiden Abschnitte 3, 4 sind jeweils im oberen Bereich massiv ausgebildet und berühren sich im zur Abschlussvorrichtung 2 eingesteckten Zustand mit der ebenen Trennfläche.

Die Abschlussvorrichtung 2 besitzt im unteren Bereich, wie in der Schnittansicht in Fig. 6 und in der Unteransicht des Gleitschutzes 1 in Fig. 7 ersichtlich, einen hohlzylindrisch ausgebildeten Aufsteck-Anlageabschnitt 12 zum Aufstecken des Gleitschutzes 1 auf einen Gehäusekopf 21 eines intraluminalen Instrumentes 20.

Der Aufsteck-Anlageabschnitt 12 besitzt eine kreisförmige Anlagefläche 13 für eine Anlage an die Stirnseite des Gehäusekopfes 21. Die kreisförmige Anlagefläche 13 weist mittig eine konisch zulaufende Spreizstift-Führungsausnehmung 14 auf.

Da sowohl die Abschlussvorrichtung 2 als auch die Anlagefüße 10 aus einem elastisch verformbaren Material hergestellt sind, kann die Abschlussvorrichtung 2 auf Gehäuseköpfe 21 mit unterschiedlichen Durchmessern angebracht werden.

An der Außenseite der beiden Abschnitte 3, 4 sind vorteilhaft im unteren Bereich jeweils zwei Zugeinrichtungs-Befestigungseinrichtungen 7 vorgesehen, die in dem vorliegenden Ausführungsbeispiel als versenkte Befestigungsösen ausgebildet sind. An diesen Befestigungsösen 7 ist jeweils eine reißfeste Schnur 8 angeknotet.

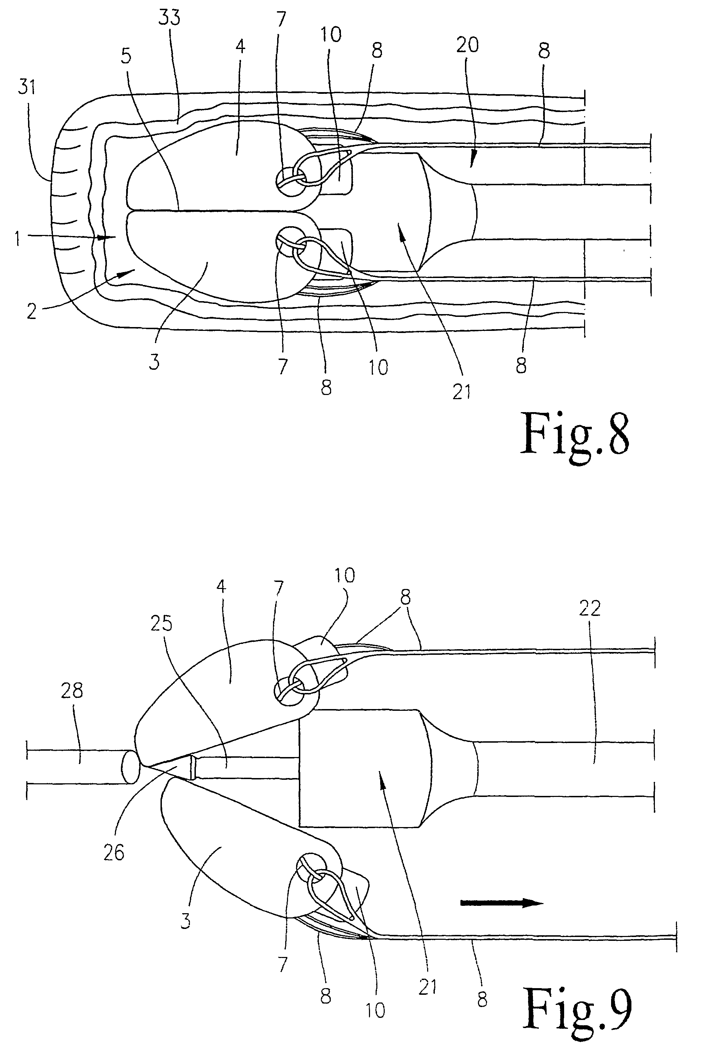

Im Folgenden soll die Vorgehensweise einer Resektion- oder Anastomoseoperationen mittels einem intraluminalen Instrument 20 und einem aufgesetzten Gehäusekopf 21 aufgesteckten Gleitschutz 1 unter Bezugnahme auf die Figuren 8 und 9 beschrieben werden.

Der Gleitschutz 1 wird vor einem Einführen des intraluminalen Instruments 20 auf den Gehäusekopf 21 des Instruments 20 aufgesteckt. Dies kann aufgrund der elastischen Eigenschaften mit der Abschlussvorrichtung 2 und der Anlagefüße 20 auf Gehäuseköpfen 21 mit verschieden großen Durchmessern ausgeführt werden. Dabei sitzt die Anlagefläche 13 des Aufsteck- Anlageabschnitt 12 flächig auf der Stirnseite des Gehäusekopfes 21. Die beiden Anlagefüße 10 der Abschlussvorrichtung 2 umschließen teilweise den äußeren Umfang des Gehäusekopfes 21.

Nach einem Einfädeln der Andruckeinrichtung 27 des intraluminalen Instruments 20 in den proximalen Darmabschnitts 30, wie in Fig. 2 ersichtlich, führt der Operateur das intraluminale Instrument 20 mitsamt Gehäusekopf 21 in den Anus ein und schiebt das Instrument 20 bis zu dem zu anastomisierenden Darmabschnitt (i. d. Regel den Rektumstumpf) vorsichtig vorwärts. Aufgrund der abgerundeten Form des Gleitschutz 1, die gleichzeitig als Abdeckung der Gehäusekopfausnehmung dient, werden keine Schleimhautfalten mitgeschoben, gedehnt und somit verletzt. Ausserdem ist die Abschlussvorrichtung 2 am Gehäusekopf 21 überkragend befestigt, wodurch der scharfkantige Rand des Gehäusekopfes 21 für eine Vermeidung von Verletzungen oder dergleichen schützend abgedeckt wird.

Beim Einführen und Vorschieben des Instruments 20 muss vom Operateur darauf geachtet werden, dass die reißfesten Schnüre 8 aus dem Anus zur jeder Zeit heraushängen und sich im Inneren des Darms nicht miteinander verknoten.

Fig. 8 zeigt eine schematische Ansicht eines bis zum neu zu anastomisierenden Darmabschnitt eingefädelten Gehäusekopf 21 mit aufgesteckten Gleitschutz 1. Die Schnüre 8 verlaufen von den Befestigungsösen 7 ausgehend in Richtung des distalen Ende des Darms.

Als nächstes fährt der Operateur, wie in Fig. 9 dargestellt, mittels einer im Gehäusegriff angebrachten Dreheinrichtung den Verbindungsstift 25 aus dem Inneren des Gehäusekopfs 21 heraus. Die Spitze 26 des Verbindungsstifts 25 gelangt zunächst in die Spreizstift-Führungsausnehmung 14 in der Anlagefläche 13 der Abschlussvorrichtung 2 und drückt bei weiterem Herausdrehen die beiden Abschnitte 3 und 4 auseinander. Der Druck, der dabei ausgeübt wird, ist ausreichend groß zu wählen, um die Verbindung mittels der Steckrücken 5 der beiden Abschnitte 3 und 4 zu trennen. Somit zerfällt bei genügend weitem Herausdrehen des Verbindungsstifts 25 der Gleitschutz 1 in zwei gleich ausgebildete Abschnitte 3 und 4.

Diese beiden Abschnitte 3 und 4 können durch ein Ziehen der Schnüre 8 problemlos nacheinander aus dem Darmabschnitt entfernt werden. Da der Darm in radialer Richtung leicht dehnbar ist, rutschen die beiden Abschnitte 3 und 4 jeweils mittels einer Zugkraft zwischen dem Gehäusekopf 21 und der Innenwand des Darmabschnitts hindurch und gleiten in Richtung des Anus. Um noch bei dieser Bewegungsrichtung eine Verletzung des Darms bzw. der Darmschleimhaut zu vermeiden, sind sämtliche Kanten, beispielsweise der Anlagefüße 10, abgerundet ausgebildet.

Durch das Entfernen des Gleitschutzes 1 vom Gehäusekopf 21 und aus dem Anastomoseabschnitt kann der Operationsvorgang gemäß der Beschreibungseinleitung in herkömmlicher Weise ausgeführt werden.

Nach einem zweiten Ausführungsbeispiel der vorliegenden Erfindung verläuft die Verbindungseinrichtung 5 entlang der rotationssymmetrischen Achse der Abschlussvorrichtung 2. Somit trennt der herausgedrehte Verbindungsstift 25 direkt die vorteilhaft als Steckbrücke ausgebildete Sollbruchstelle.

Mittels einem dritten Ausführungsbeispiel ist die Abschlussvorrichtung 2 nicht homogen aus dem selben elastisch verformbaren Material gebildet, sondern als Hohlkörper ausgebildet, der mit einem geeigneten, in der inerten Material ausgefüllt ist.

Obwohl die vorliegende Erfindung anhand bevorzugter Ausführungsbeispiele vorstehend beschrieben wurde, ist sie darauf nicht beschränkt, sondern auch vielfältigerweise modifizierbar.

Beispielsweise können die beiden Abschnitte 3 und 4 nur in einem kleinen Teilbereich in einer aneinander grenzenden Fläche mittels einer Verbindungseinrichtung verbunden sein.

Mit der vorliegenden Erfindung wird somit ein Gleitschutz geliefert, mit dem medizinische Instrumente ohne Verletzung des Inneren eines Hohlorgans oder der darin enthaltenen Schleimhaut in die entsprechende Abschnitte eingeführt und vorwärts geschoben werden können. Dabei kann der Gleitschutz von Operateur von außen vom jeweiligen Gehäusekopf entfernt und aus dem entsprechenden Abschnitt gezogen werden. Somit behindert der Gleitschutz nicht den weiteren entscheidenden Operationsvorgang, d.h. die Darmanastomose.

Außerdem geraten vorteilhaft keine Kunststoffanteile der Abschlussvorrichtung in dem Schneid- und Klammermechanismus, da die Abschlussvorrichtung außen um Gehäusekopf angebracht ist.

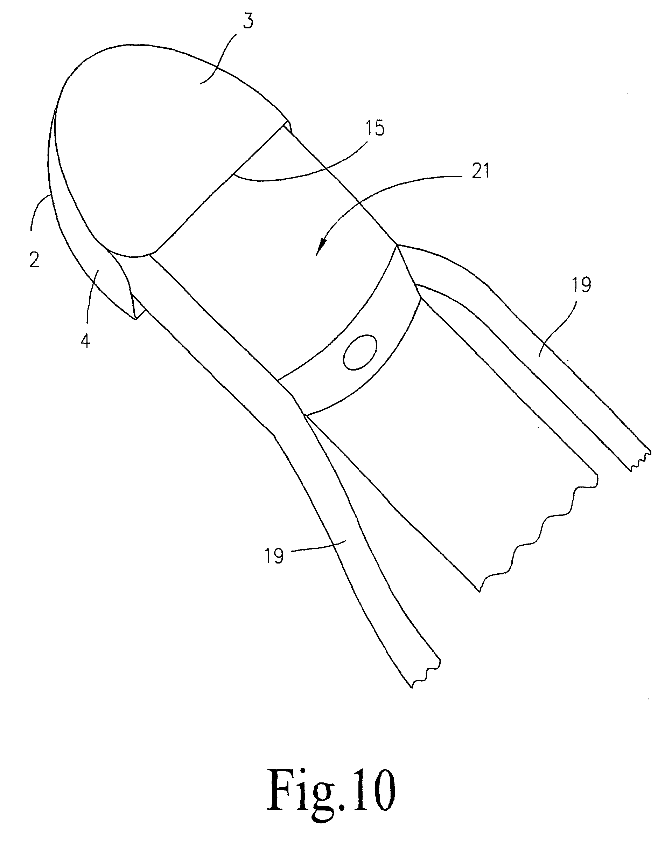

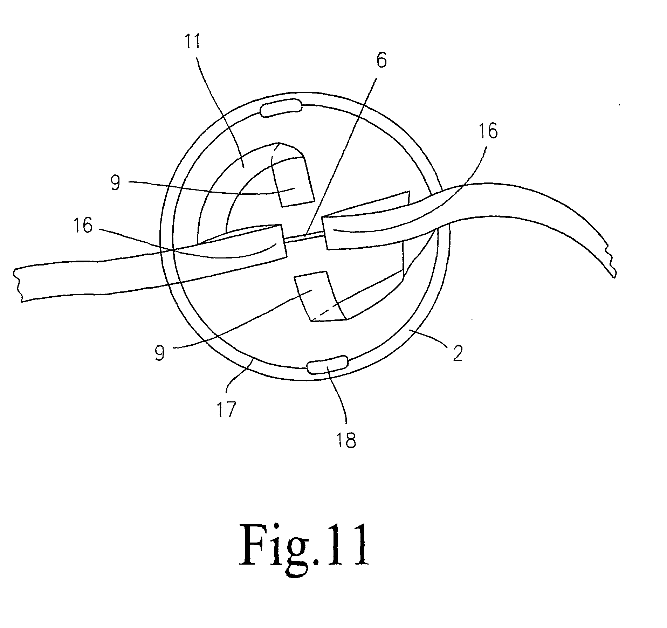

In Fig. 10 ist ein weiteres Ausführungsbeispiel eines erfindungsgemäßen Gleitschutzes in Anordnung auf einem Gehäusekopf 21 dargestellt. Bei diesem Gleitschutz sind zwei eiförmige Kappenteile 3 und 4 vorgesehen, die die Abschlussvorrichtung 2 bilden. Die beiden Kappenteile 3 und 4 werden, wie in Fig. 11 angedeutet, durch Abschnitte zweier Zugbänder 19 an ihrer Nahtstelle 6 verbunden. Die Verbindung erfolgt dabei durch einen lösbaren Kleber, wobei das Zugband auf der Innenfläche der Kappenteile zweimal annähernd rechtwinklig gefaltet ist und schließlich fest im Innern des Kappenteils senkrecht zur Nahtstelle 6 befestigt ist. Die rechtwinkligen Bandhaltungen sind ebenfalls mit einem lösbaren Kleber vorgenommen.

Jedes Kappenteil 3, 4 besitzt an dem halbkreisförmigen unteren Abschluss eine Anlagekante, die in der Anlagestellung der Abschlussvorrichtung 2 den äußeren Umfang des Gehäusekopfes 21 umschließt. An der Innenseite jedes Kappenteiles ist eine Nut 17 eingefräst, mit der sich jedes Kappenteil am Rand des Gehäusekopfes 21 abstützt. Um 90° C zur Naht 6 versetzt ist bei jedem Kappenteil 3, 4 eine randseitige Zugband-Führungseinformung 18 nahe seiner endseitigen Befestigung vorgesehen. Die Führungseinformung ist als flache Nut ausgebildet, deren Breite etwa der des flachen Zugbandes 19 entspricht.

Nach dem Aufsetzen der Abschlussvorrichtung 2 aus dem in Fig. 11 dargestellten Zustand in die Position gemäß Fig. 10 auf dem Gehäusekopf 21 ragen die Zugbänder 19 aus der eikappenförmigen Abschlussvorrichtung 2 hervor und werden in Richtung Griff des medizinischen Instruments geführt.

Nach dem beschriebenen Einführen des Instruments 20 durch den Analkanal in den zu anastomisierenden Rektumanteil, wird nach Erreichen der gewünschten Position an jedem Zugband 19 gleichzeitig ein Zug in Richtung Griff ausgeübt, wodurch sich das in der Kappeninnenfläche gewinkelt zusammengelegte Zugband entfaltet und an der Innenfläche des jeweiligen Kappenteils in Richtung der flachen randseitigen Führungseinformung 18 rutscht. Dabei wird das leichte Entfalten des Zugbandes durch die leichte Lösbarkeit der Klebeverbindung an der Nahtstelle 6 der beiden Kappenteile sowie der leichten Löslichkeit der mit Kleber fixierten Abwinklungen des Zugbandes erreicht.

Durch Ziehen an den Zugbändern 19 in Richtung Griff werden die beiden Kappenteile aufgefaltet und am Gehäusekopf 21 entlang in Richtung Griff geführt. Dabei sind die beiden Kappenteile so geformt, dass sie am Schaft des eingeführten Instruments Entlanggleiten können und kein Hindernis beim Entfernen des Instruments aus dem Arnus darstellen. Der Operateur kann nun, wie beschrieben, die Darmanastomose durchführen.

### Bezugszeichenliste:

- 1.: Gleitschutz
- 2.: Abschlussvorrichtung
- 3.: Erster Abschnitt
- 4.: Zweiter Abschnitt
- 5.: Steg- bzw. Steckbrücke
- 6.: Nahtstelle Kappenteile Halbschalen
- 7.: Befestigungsöse
- 8.: Zugeinrichtung
- 9.: Feste Zugbandverankerung oberer Pol Halbschale
- 10.: Anlagefuß
- 11.: Halbkreisförmige Bandführung
- 12.: Aufsteck-Anlageabschnitt
- 13.: Anlagefläche
- 14.: Spreizstift-Führungsausnehmung
- 15.: Abgeflachtes Ende
- 16.: Gefalteter Zugbandanteil auf Nahtstelle 6 löslich geklebt
- 17.: Anlageaufformung bzw. Anlagekante Halbschale
- 18.: Führungseinformung
- 19.: Zugbandführung nach Aufsteckung der Abschlussvorrichtung
- 20.: intraluminales Instrument
- 21.: Gehäusekopf
- 22.: Gehäuseschaft
- 23.: Griff
- 24.: Dreheinrichtung
- 25.: Verbindungsstift
- 26.: Spitze des Verbindungsstiftes
- 27.: Andruckeinrichtung
- 28.: Aufnahmehülse
- 29.: Schneid- und Klammermechanismus
- 30.: Proximaler Darmabschnitt
- 31.: Distaler Darmabschnitt
- 32.: Ausgestanzte Gewebering
- 33.: Schleimhaut
- 34.: Auslösehebel

## Patentansprüche

1. Gleitschutz (1) für einen Gehäusekopf (21) medizinischer Instrumente (20), insbesondere intraluminaler Instrumente (20) für Resektions- oder Anastomoseoperationen, mit einer am Gehäusekopf (21) überkragend befestigbaren Abschlussvorrichtung (2), die fernbetätigbar in wenigstens zwei Abschnitten (3,4) vom Gehäusekopf (21) entfembar ist.

2. Gleitschutz nach Anspruch 1, **dadurch gekennzeichnet, dass** die Abschlussvorrichtung (2) rund, insbesondere eiförmig, mit einem einseitig abgeflachten Ende (15) ausgebildet ist.

3. Gleitschutz nach einem der Ansprüche 1 oder 2, **dadurch gekennzeichnet, dass** die Abschlussvorrichtung (2) aus einem elastisch verformbaren Material hergestellt ist.

4. Gleitschutz nach einem der vorhergehenden Ansprüche, **dadurch gekennzeichnet, dass** die Abschlussvorrichtung (2) an jedem der wenigstens zwei Abschnitte (3,4) mindestens eine Zugeinrichtungs-Befestigungseinrichtung (7) aufweist.

5. Gleitschutz nach Anspruch 4, **dadurch gekennzeichnet, dass** an jeder Zugeinrichtungs-Befestigungseinrichtung (7) eine fernbetätigbare Zugeinrichtung (8), insbesondere eine reißfeste Schnur (8), anbringbar ist.

6. Gleitschutz nach Anspruch 4 oder 5, **dadurch gekennzeichnet, dass** mindestens eine Zugeinrichtungs-Befestigungseinrichtung (7) als versenkte Befestigungsöse ausgebildet ist.

7. Gleitschutz nach einem der vorhergehenden Ansprüche, **dadurch gekennzeichnet, dass** die wenigstens zwei Abschnitte (3,4) symmetrisch ausgebildet sind.

8. Gleitschutz nach einem der vorhergehenden Ansprüche, **dadurch gekennzeichnet, dass** die wenigstens zwei Abschnitte (3,4) über mindestens eine Verbindungseinrichtung (5), insbesondere als stegförmige Steckbrücke (5) ausgebildete Sollbruchstelle, miteinander verbindbar sind.

9. Gleitschutz nach einem der vorhergehenden Ansprüche, **dadurch gekennzeichnet, dass** die wenigstens zwei Abschnitte (3,4) jeweils flache Bereiche aufweisen, die bei einer Verbindung der wenigstens zwei Abschnitte (3,4) abschließend aneinanderliegen.

10. Gleitschutz nach einem der vorhergehenden Ansprüche, **dadurch gekennzeichnet, dass** jeder der wenigstens zwei Abschnitte (3,4) am abgeflachten Ende (15) mindestens einen Anlagefuß (10) aufweist, der abschließend teilweise den äußeren Umfang des Gehäusekopfes (21) umschließt.

11. Gleitschutz nach Anspruch 10, **dadurch gekennzeichnet, dass** die Anlagefüße (10) elastisch verformbar ausgebildet sind.

12. Gleitschutz nach einem der vorhergehenden Ansprüche, **dadurch gekennzeichnet, dass** das abgeflachte Ende (15) der Abschlussvorrichtung (2) einen hohlzylindrisch ausgebildeten Aufsteck- Anlageabschnitt (12) aufweist.

13. Gleitschutz nach Anspruch 12, **dadurch gekennzeichnet, dass** der Aufsteck-Anlageabschnitt (12) eine kreisförmige Anlagefläche (13) mit einer mittig angeordneten, konisch zulaufende Spreizstift- Führungsausnehmung (14) aufweist.

14. Gleitschutz nach einem der Ansprüche 1 bis 7, **dadurch gekennzeichnet, dass** die beiden Abschnitte als abgerundete Kappenteile (3, 4) ausgebildet und miteinander über lösbare Zugbandbefestigungen (16) verbunden sind.

15. Gleitschutz nach Anspruch 14, **dadurch gekennzeichnet, dass** das Ende des Zugbandes (19) an jedem Kappenteil (3, 4) separat befestigt ist, wobei das Zugband (19) zwischen seiner endseitigen Befestigung (9) und der die Kappenteile (3, 4) verbindenden Befestigung (16) wenigstens einmal annähernd rechtwinklig gegen die Innenfläche des Kappenteils gefaltet ist.

16. Gleitschutz nach Anspruch 14 oder 15, **dadurch gekennzeichnet, dass** jedes Kappenteil (3, 4) eine randseitige Zugband-Führungseinformung (18) nahe seiner endseitigen Befestigung (9) aufweist.

17. Gleitschutz nach einem der Ansprüche 14 bis 16, **dadurch gekennzeichnet, dass** jedes Kappenteil (3, 4) eine Anlageeinformung (17) für den Gehäusekopf (21) aufweist.

## Claims

1. Anti-slip protection device (1) for a housing head (21) for medical instruments (20), in particular intraluminal instruments (20) for resection or anastomosis operations, comprising a terminal device (2) which can be fastened to the housing head (21) so as to project out, the terminal device being removable from the housing head (21), in a remotely controllable manner, in at least two sections (3, 4).

2. Anti-slip protection device according to claim 1, **characterised in that** the terminal device (2) is round in design, in particular ovoid, with one end (15) flattened on one side.

3. Anti-slip protection device according to either of claims 1 or 2, **characterised in that** the terminal device (2) is produced from an elastically deformable material.

4. Anti-slip protection device according to any one of the preceding claims, **characterised in that** the terminal device (2) has at least one pull mechanism/fastening mechanism (7) on each of the at least two sections (3, 4).

5. Anti-slip protection device according to claim 4, **characterised in that** a remotely actuable pull mechanism (8), in particular a tear resistant cord (8) can be provided on each pull mechanism/fastening mechanism (7).

6. Anti-slip protection device according to claim 4 or 5, **characterised in that** at least one pull mechanism/fastening mechanism (7) is formed as a recessed fastening eyelet.

7. Anti-slip protection device according to any one of the preceding claims, **characterised in that** the at least two sections (3, 4) are formed symmetrically.

8. Anti-slip protection device according to any one of the preceding claims, **characterised in that** the at least two sections (3, 4) can be connected to one another, in particular as a rupture joint formed as a web-shaped insertion bridge (5) by way of at least one connecting mechanism (5).

9. Anti-slip protection device according to any one of the preceding claims, **characterised in that** the at least two sections (3, 4) each have flat regions which abut one another in a closing manner in the event of a connection of the at least two sections (3, 4).

10. Anti-slip protection device according to any one of the preceding claims, **characterised in that** each of the at least two sections (3, 4), at the flattened end (15), has at least one contact foot (10) which partially encloses the outer periphery of the housing head (21) in a closing manner.

11. Anti-slip protection device according to claim 10, **characterised in that** the contact feet (10) are formed so as to be elastically deformable.

12. Anti-slip protection device according to any one of the preceding claims, **characterised in that** the flattened end (15) of the terminal device (2) has a hollow-cylindrical push-on contact section (12).

13. Anti-slip protection device according to claim 12, **characterised in that** the push-on contact section (12) has a circular contact face (13) with an expansion pin-guide recess (14) tapering conically and arranged centrally.

14. Anti-slip protection device according to any one of claims 1 to 7, **characterised in that** the two sections are formed as rounded cap parts (3, 4) and are connected to one another by way of detachable pull strip fastenings (16).

15. Anti-slip protection device according to claim 14, **characterised in that** the end of the pull strip (19) is fastened separately to each cap part (3, 4), the pull strip (19) between its end fastening (9) and the fastening (16) connecting the cap parts (3, 4) being folded at least once approximately at right angles towards the internal face of the cap part.

16. Anti-slip protection device according to claim 14 or 15, **characterised in that** each cap part (3, 4) has an edge pull strip-guide mechanism (18) close to its end fastening (9).

17. Anti-slip protection device according to any one of claims 14 to 16, **characterised in that** each cap part (3, 4) has a contact moulding (17) for the housing head (21).

## Revendications

1. Dispositif de protection favorisant le glissement pour la tête formant boîtier (21) d'instruments médicaux (1), plus particulièrement d'instruments (20) intraluminaux destinés à des opérations de résection ou d'anastomose, comportant un dispositif de protection (2) qui est fixé de manière enveloppante sur la tête formant boîtier (21) et peut être éloigné de celle-ci en au moins deux parties (3, 4), d'une manière commandée à distance.

2. Dispositif de protection favorisant le glissement selon la revendication 1, **caractérisé par le fait que** le dispositif de protection (2) est rond, plus particulièrement ovoïde, avec une extrémité (15) aplatie d'un côté.

3. Dispositif de protection favorisant le glissement selon la revendication 1 ou 2, **caractérisé par le fait que** le dispositif de protection (2) est fabriqué en un matériau déformable élastiquement.

4. Dispositif de protection favorisant le glissement selon une des revendications précédentes, **caractérisé par le fait que** le dispositif de protection (2), sur chacune des parties (3, 4), au nombre d'au moins deux, comporte au moins un dispositif d'attache (7) pour un moyen de traction.

5. Dispositif de protection favorisant le glissement selon la revendication 4, **caractérisé par le fait qu'**à chaque dispositif d'attache (7) de moyen de traction est fixé un moyen de traction (8) actionnable à distance, plus particulièrement un cordon résistant à l'arrachement.

6. Dispositif de protection favorisant le glissement selon la revendication 4 ou 5, **caractérisé par le fait qu'**au moins un dispositif d'attache (7) de moyen de traction est conformé en oeillet de fixation encastré.

7. Dispositif de protection favorisant le glissement selon une des revendications précédentes, **caractérisé par le fait que** les parties (3, 4), au nombre d'au moins deux, sont symétriques.

8. Dispositif de protection favorisant le glissement selon une des revendications précédentes, **caractérisé par le fait que** les parties (3, 4), au nombre d'au moins deux, peuvent être liées l'une à l'autre par l'intermédiaire d'au moins un moyen de liaison (5), plus particulièrement par l'intermédiaire d'un point destiné à la rupture conformé en barrette (5).

9. Dispositif de protection favorisant le glissement selon une des revendications précédentes, **caractérisé par le fait que** les parties (3, 4), au nombre d'au moins deux, présentent chacune des zones planes, qui lorsque les parties (3, 4) sont liées, sont appliquées l'une sur l'autre de manière enveloppante.

10. Dispositif de protection favorisant le glissement selon une des revendications précédentes, **caractérisé par le fait que** chacune des parties (3, 4), au nombre d'au moins deux, au niveau de l'extrémité (15) aplatie, présente au moins une embase (10) qui entoure partiellement le pourtour extérieur de la tête formant boîtier (21).

11. Dispositif de protection favorisant le glissement selon la revendication 10, **caractérisé par le fait que** les embases (10) sont déformables élastiquement.

12. Dispositif de protection favorisant le glissement selon une des revendications précédentes, **caractérisé par le fait que** l'extrémité (15) aplatie du dispositif de protection (2) comporte une portion d'appui-enfichage (12) conformée en cylindre creux.

13. Dispositif de protection favorisant le glissement selon la revendication 12, **caractérisé par le fait que** la portion d'appui-enfichage (12) comporte une surface d'appui (13) circulaire avec un évidement (14) de guidage de broche expansible central, conique.

14. Dispositif de protection favorisant le glissement selon une des revendications 1 à 7, **caractérisé par le fait que** les deux parties sont conformées en élément de coiffe (3, 4) arrondis et sont liées entre elles par des liaisons à liens (16) détachables.

15. Dispositif de protection favorisant le glissement selon la revendication 14, **caractérisé par le fait que** l'extrémité du lien (19) est fixée séparément à chaque partie formant coiffe (3, 4), le lien (19) entre sa fixation (9) extrémale et la liaison (16) assurant l'assemblage des parties formant coiffe (3, 4), étant plié au moins une fois à angle droit contre la paroi intérieure de la partie formant coiffe.

16. Dispositif de protection favorisant le glissement selon la revendication 14 ou 15, **caractérisé par le fait que** chaque partie formant coiffe (3, 4), dans le voisinage de sa fixation extrémale (9), présente une échancrure (18) de guidage du lien disposée sur le bord,.

17. Dispositif de protection favorisant le glissement selon une des revendications 14 à 16, **caractérisé par le fait que** chaque partie formant coiffe (3, 4) présente une échancrure d'appui (17) pour la tête formant boîtier (21).
